# EUROPEAN PATENT SPECIFICATION

(11) **EP 1 003 970 B1**
(45) Date of publication and mention of the grant of the patent: **25.08.2004**
(21) Application number: 98932850.5
(22) Date of filing: 25.06.1998
(51) Int. Cl.: F04B 35/04, F04D 13/06, A61M 1/10

(54) **IMPROVED ROTOR FOR BLOOD PUMP**
ROTOR FÜR EINE BLUTPUMPE
ROTOR AMELIORE POUR POMPE A SANG

(30) Priority: 01.07.1997 US 886185; 01.07.1997 US 886188; 01.07.1997 US 886385
(43) Date of publication of application: 31.05.2000
(73) Proprietor: Advanced Bionics, Inc., Hopkins, MN 55343 (US)
(72) Inventor: IZRAELEV, Valentin, M., Eden Prairie, MN 55344 (US)
(74) Representative: Powell, Timothy John
(86) International application number: PCT/US1998/013184
(87) International publication number: WO 1999/001663

(56) References cited:
- DE-A- 19 613 388
- US-A- 4 507 048
- US-A- 4 944 748
- US-A- 5 055 005
- US-A- 5 385 581
- US-A- 5 685 700

## Description

### BACKGROUND OF THE INVENTION

The present invention relates generally to an improved rotor structure for implementation as a pump for transferring fragile or aggressive fluids, and with the rotor having radial vanes thereon and in certain embodiments, the vanes may enclose and/or encapsulate magnetic drive components. Examples of fragile fluids include human or animal blood, neither of which can tolerate exposure to unusual impact and/or sheer forces. Aggressive fluids include corrosive or poisonous fluids, as well as fluids which cannot tolerate contamination, or which otherwise may destroy seals and/or bearings to reduce the lifetime and/or longevity of the pump structure. Poisonous fluids, for example, are extremely dangerous if a leak develops. More particularly, the present invention relates to a rotor for a pump which is bearing and seal-free and wherein the rotor has a core body with one or more shrouds coupled to the outer surface of the core and arranged in parallelly disposed relationship with the outer surface of the core. In addition, the rotor is dynamically balanced by a combination of hydrodynamic and buoyant forces. In this configuration, the design of the rotor provides a plurality of parallelly arranged flow channels through which a fluid contacting area is provided for enhancing flow. In the other configuration, the vanes are presented between the core and the shroud, or between the shrouds for enhancing flow. In the configuration utilizing multiple shrouds, a primary flow channel will be created between the shroud and the core, with a secondary flow channel being arranged outside of the shroud. The primary channel provides meridional channels, while the secondary flow channel provides communication for flow between the inlet and the outlet.

The pump of the present invention is particularly adapted for transferring human blood and is capable of creating a flow of such liquids without damaging and/or otherwise adversely affecting the quality of the material being pumped. The rotor employed in the pump of the present invention including its multiple flow channels is rotated electromagnetically by means of an electromagnetic drive system operating in conjunction with one or more arrays of permanent magnets preferably enclosed within radial vanes on the surface of the rotor, the drive components being arranged in a brushless motor configuration. Alternatively, a permanent magnet-to-permanent magnet coupling may be employed. As such, the arrangement of the present invention provides a symmetrical arrangement which is capable of achieving relative rotation while at the same time being bearing and seal-free.

In the past, pumps and pumping systems have been designed which have been characterized as being bearing and seal-free. Such systems typically employ magnetic levitation means which is in effect an actual form of bearing, much the same as sleeve bearings, ball bearings, or other friction-inducing bearings. Such arrangements using magnetic bearings, while being operational and functional, may be rendered complex and accordingly require significant number of additional components including magnetic devices, position sensors, and rapid-response magnetic drive means. A number of such patents have been granted in the past, including those to Olsen et al. 4,688,998 and 5,195,877. The apparatus of the present invention, by contrast, is fully bearing and seal-free, and is provided with a rotor having a central internal bore to accommodate fluid flow input, as well as externally-positioned annular channels for accommodating flow through the pump structure with dynamic balance being achieved through a combination of hydrodynamic and buoyant forces. The rotor of the pump of the present invention is provided with a symmetrical arrangement of radial vanes which preferably enclose components of the magnetic drive so as to enhance and preserve the dynamic balance.

Among the disadvantages inherent in pumps utilizing friction-reducing bearings include local heat generation such as may occur from the use of ball bearings, friction bearings, sleeve bearings, and the like. Low flow and high pressure may result in local areas due to the use of such structures. In addition, with all such bearing-equipped pumps, a high spring constant is provided wherein a small displacement of the rotor (or impeller) introduces very high forces which can damage or effectively destroy bearings. In addition, different forces are introduced in the structure whenever variations in axial positions occur.

US patent No. 5,055,005 discloses a fluid pump with rotary impeller, which rotary impeller comprises an electro-magnetically driven rotary impeller levitated by localised opposed fluid forces. Levitation by localised opposed fluid forces of an impeller driven by electromagnetic forces eliminates the need for bearings and seals in the driving mechanism. This avoids the heat build-up and leakage associated with other pumping mechanisms, which can be of importance for pumping of physiological fluids such as blood.

US patent No. 5,385,581 discloses an impeller forming part of a blood pump which is supported by permanent magnets on the impeller and pump housing and stabilised by an electromagnet on the housing. A control circuit supplies current to the electromagnet to maintain the axial position of the impeller at a control position in which the impeller is in mechanical equilibrium under permanent magnet forces and static axial forces on the impeller to minimise energy consumption in the support of the impeller. The impeller is rotated magnetically and stator coils in the housing are supplied with electric current having a frequency and amplitude adjusted in relation to blood pressure at the pump inlet to match the flow characteristics of the pump to physiological characteristics of the natural heart. A cavity is formed in the impeller to match the average specific gravity of the impeller and portions of the suspension and drive systems thereon to the specific gravity of blood to further minimise power consumption by the pump. A valve member can be formed on the impeller to mate with a restriction in the pump inlet for pumps used to assist the pumping action of the natural heart.

According to a first aspect of the invention there is provided a pump as defined in Claim 1.

In the present structure, the pump is bearing and seal-free, with the structure of one embodiment of the invention including a rotor having an internal bore for accommodating inlet fluid, and is further provided with a series of external shrouds providing multiple annular flow channels, with this design allowing for relatively high displacement without the creation of large forces otherwise required to hold the rotor in its predetermined position. The internal bore which may be formed within the rotor is arranged coaxially with the rotor and thus fluid flow is readily accomplished therewithin. In addition, the rotor seeks and finds an equilibrium position during operational rotation which, in certain situations, can be off-set from the housing axis (in either the rotational or transverse axes) which typically occurs when the rotational axis of the pump is altered. Rotational movement of the pump housing will be manifested in displacement of the rotational or vertical axis of the rotor. The present arrangement has been found to eliminate the need for a highly precise axis in design, fabrication and operation. The lack of a positionally fixed rotational axis reduces the introduction of large forces which otherwise would be created when the axis of the rotor is shifted away from its normal centrally disposed position.

In addition to the outer surface of the rotor core, one or more shrouds are arranged concentrically with the outer surface of the rotor core, with the configuration providing one or more annular channels for flow. In addition to this, introduction of the vanes acting as paddles between the core and the shroud or in the case of multiple shrouds, then between the shrouds, with this arrangement providing even more channels for flow through the rotor.

In one embodiment of the present invention, the pump includes a pumping chamber with a central axis, and with a rotor body being disposed within the chamber for bearing and seal-free rotation therewithin. The rotor has a core with a double or dual-conical configuration which converges toward opposed polar regions, and with the axis of rotation extending between these polar regions.

In addition to the rotor core, one or more concentric shrouds are provided to increase the area of contact between the fluid being pumped and the surface of the rotor, and to provide annular channels through which fluid flow may occur.

The rotor may be further provided with radial vanes extending radially outwardly relative to the axis of rotation. These vanes are utilized to enhance flow, as well as to provide a zone for encapsulation of magnetic drive components. Magnetic drive components are typically arranged symmetrically in axially spaced-apart relationship relative to the transverse axis of the rotor.

A fluid inlet port is preferably arranged in the pumping chamber with a bore coaxially with the axis of rotation of the rotor in order to provide for inlet flow to opposed ends of the rotor. In this arrangement, the housing inlet flow is divided into two approximately equal flow portions, with the first flow portion entering the rotor at the end adjacent the housing inlet or the external inlet, and the second flow portion being drawn through the bore to the housing end opposite to the external inlet where the second flow portion makes a smooth reversal of direction and enters the rotor at the opposed end. Accordingly, one portion of the flow of fluid is transported or transferred to the portion of the rotor which is in opposed polar relationship to the housing inlet port, in other words, the external inlet.

Except for those occasions when the rotor is displaced, it is normally arranged in coaxial relationship with both the pumping chamber and the fluid inlet ports. The outlet port or ports are arranged generally medially of the chamber, midway between the inlet ports and typically are positioned tangentially of the medial portion of the pumping chamber. In those situations where the axis of rotation of the rotor is arranged vertically, the dual-conical configuration is such that flow through the bore of the rotor and on the outside surface of the rotor core and in the annular channels proceeds downwardly on the upper portion, and upwardly on the lower portion of the dual-cone.

The rotor of at least one embodiment provides for an internal transfer of fluids between the oppositely disposed fluid inlet areas with the rotor bore forming a fluid transfer line which introduces the fluids to the rotor at opposite ends of the housing. The bore provides communication between opposite ends of the rotor, thereby permitting transfer of fluids internally of the structure, with all of the fluid being initially introduced into one polar region of the housing. The fluid is thereafter transferred internally to the oppositely disposed polar region.

The term "oppositely disposed inlet ports" is intended to reflect the utilization of fluid introduction at opposite ends of the rotor, and is also intended to include those arrangements wherein all of the fluid being pumped is initially introduced into one polar region of the housing, with the fluid nevertheless being transferred either internally or externally of the housing directly to the oppositely disposed polar region.

The pump shown in the drawings is in operational mode with the rotor spinning about its axis of rotation and with all forces acting on the rotor balanced. In the stationary/non-operational mode with the fluid in the housing, only the buoyant forces are acting on the rotor, and the rotor floats up in the random position. In the stationary/non-operational mode with no fluid in the housing, the rotor is resting on the interior of the housing under gravitational forces.

Levitation of the rotor, as indicated, is achieved by a combination of hydrodynamic and buoyant forces. Briefly, the buoyant component is achieved as a result of careful selection of the rotor density, with the preferred relative density being between about 0.1 and 0.9 of the relative density of the fluid being pumped. The term "relative density" as will be appreciated, defines the density of the rotor which is measured relative to the density of the fluid being pumped. In a dynamic and operational mode, the buoyant forces merely become a component of lesser or secondary importance to the more significant and more highly effective hydrodynamic force.

The hydrodynamic force component is achieved as a result of the motion of the fluid as it is being moved through the pumping chamber. As the velocity of the fluid increases, the hydrodynamic forces increase substantially, and with the proper selection of rotor density, the hydrodynamic forces which are created during normal operation result in achieving a precise, steady and controllably repeatable centering of the rotor within the pumping chamber.

The intent of the present invention is to bring the fluid from the opposite inlet regions of the housing to the medial plane of the housing, combine two opposite flows in the medial plane, and deliver the fluid to the outlet port with a minimal damage and losses by avoiding turbulence, flow separation, sharp turns, stagnation, and other undesired conditions. This is achieved by having the main flows through the rotor channels, secondary flows between the inner periphery of the housing, and the outer periphery of the rotor shroud, bringing into coincidence the medial planes of the housing and the rotor, and by moving away the electromagnetic drive means plane from the median planes of the housing and the rotor to provide for improved coupling and flow.

The pump structure of the present invention has particular application for transferring fragile and/or aggressive liquids, in particular, for transferring human blood. Since certain components in blood are extremely fragile and are damaged upon exposure to external forces, conventional pumps are simply unsuited for the application. Additionally, conventional seals and/or bearings typically found within conventional pump structures pose substantial and significant threats to cell damage. A further feature of the pump of the present invention rendering the pump well suited for transfer of blood is its essentially friction-free operation. Any frictional force due to relative motion between the rotor and the stator creates the risk of generation of thermal energy, and thus may contribute to heat build-up. Since blood is extremely sensitive to temperature change, particularly any increase in temperature above conventional body temperature, reduction and/or virtual elimination of friction provides significant and substantial advantages.

Since the structure of the present invention does not require bearings, energy consumption is reduced through the elimination of energy losses otherwise occurring in the bearings, including energy lost in contact bearings as well as electrical losses in magnetic bearings. The driving forces for the impeller may be located generally in the plane of the center of gravity or center of mass of the impeller, or adjacent thereto and normal to the axis of rotation. This feature results in the creation of a gyroscopic effect of a free-body gyroscope, and the configuration of the present invention is such as to stabilize the impeller when the axis of the housing is rotated relative to the spin axis of the rotor. In other words, the spin axis of the rotor may be altered because of a change-of-position of the housing, and thus the spin axis may not always be about the vertical axis, but can be about the horizontal axis as well.

In at least one embodiment of the present invention, the magnetic drive components are offset from the transverse axis of the rotor, and in this configuration, it has been found that the center of gravity or center of mass of the rotor is generally displaced from the geometric center in a direction toward the mounting point of the drive.

In addition to blood pump applications, the device of the present invention finds utility in connection with other fluids as well. Certainly non-delicate fluids may be appropriately treated and/or moved with pump devices of the present invention including the aggressive fluids as discussed hereinabove. Eliminating shafts, bearings and seals substantially reduces the manufacturing cost of the present pump. Also, the present pump has a virtual unlimited mechanical life under normal conditions. The device of the present invention finds utility for any fluids when economy, longevity, and uninterrupted service are the factors.

A feature of one embodiment of the present invention is to bring the fluid from the opposite inlet regions of the housing to the medial plane of the housing, combine in the medial plane two opposite flows, and deliver the fluid to the outlet port or ports with minimal damage and losses to the fluid being pumped through avoidance of turbulence, flow separation, sharp turns, stagnation, and other undesired conditions. This is achieved by combining the upper and lower pair of respective vanes in the single unit at the outlet region, encapsulating the permanent magnets into the vanes at the outer tips of the vanes, and by moving the electromagnetic drive means from the median plane of the housing and the rotor.

Also in accordance with the present invention, only one inlet to the housing is provided, and correspondingly only one line of plumbing coupled to the pump is required. Offsetting the plane of the drive means from the outlet/medial plane of the housing further makes the pump design, operation, and maintenance more convenient and allows one to use a conventional drive means, especially in the permanent magnet-to-permanent magnet configuration.

### SUMMARY OF THE INVENTION

Therefore, it is a primary advantage of the present invention to provide an improved pump for transferring fragile liquids such as human blood, and wherein the pump is bearing and seal-free, with the rotor having at least one shroud in spaced relationship to the outer surface of the rotor core to create one or more annular flow channels, and with the rotor having radial vanes which may enclose magnetic drive components adjacent the radial outer ends thereof, and with the rotor being dynamically balanced upon rotation by a combination of hydrodynamic and buoyant forces, and preferably having an internal bore formed therewithin to transfer incoming fluid from one polar end of the rotor to the other.

It is yet a further advantage of the present invention to provide an improved pump for application with human blood which is capable of creating a uniform and consistent flow of such liquids without damaging or otherwise adversely affecting the quality of the material being pumped.

It is yet a further advantage of the present invention to provide a pump structure utilizing a pumping chamber housing a shrouded rotor wherein rotation of the rotor is achieved by an electromagnetic drive system operating in conjunction with an array of permanent magnets distally positioned in radial vanes mounted on the rotor and arranged on the rotor in a brushless configuration.

Other and further advantages of the present invention will become apparent to those skilled in the art upon a study of the following specification, appended claims, and accompanying drawings.

### IN THE DRAWINGS

Figure 1 is a perspective view of a pump assembly prepared in accordance with the present invention;
Figure 2 is a vertical sectional view taken through the axis of the structure as illustrated in Figure 1, and illustrating the configuration of the rotor including the rotor core and shroud, and with this view further illustrating the flow pattern created by the pump when in actual operation;
Figure 3 and 4 are horizontal sectional views taken along the line and in the direction of the arrows 3-3 and 4-4, respectively, of Figure 2;
Figure 5 is a view similar to Figure 2, and illustrating a modified configuration for the drive components;
Figure 6 is a view similar to Figure 2 and illustrating a modified shroud configuration for the rotor;
Figure 7 is a fragmentary sectional view taken on a slightly enlarged scale and illustrating the configuration of the clearance between the rotor and housing;
Figure 8 is a schematic diagram illustrating a typical system in which the device of the present invention may function;
Figure 9 is a perspective view of an alternate preferred embodiment of a pump assembly prepared in accordance with the present invention;
Figure 10 is a vertical sectional view of the assembly and taken through the axis of rotation of the rotor portion of the structure as illustrated in Figure 9, with Figure 10 being taken along the line and in the direction of the arrows 10-10 of Figure 11, and further illustrating the arrangement of the drive components of the pump;
Figure 11 is a horizontal sectional view of the pump taken along the line and in the direction of the arrows 11-11 of Figure 10;
Figure 12 is a fragmentary sectional view taken on a slightly enlarged scale and illustrating the clearance between the rotor and housing, and illustrating the manner in which a portion of the fluid flows through the assembly;
Figure 13 is a schematic diagram illustrating a typical system in which the device of the present invention may function;
Figure 14 is a perspective view of an additional alternate preferred embodiment of a pump assembly prepared in accordance with the present invention;
Figure 15 is a vertical sectional view taken through the axis of the rotor, and illustrating the flow patterns created by the pump when in actual operation;
Figures 16 and 17 are horizontal sectional views of the pump structure illustrated in Figure 15, and taken along the lines 16-16 and 17-17 respectively of Figure 15;
Figure 18 is a fragmentary view on an enlarged scale and illustrating the magnitude of the clearance between the exterior of the rotor and the interior of the housing; and
Figure 19 is a schematic diagram illustrating a typical system in which the device of the present invention may function.

### DESCRIPTION OF THE PREFERRED EMBODIMENT

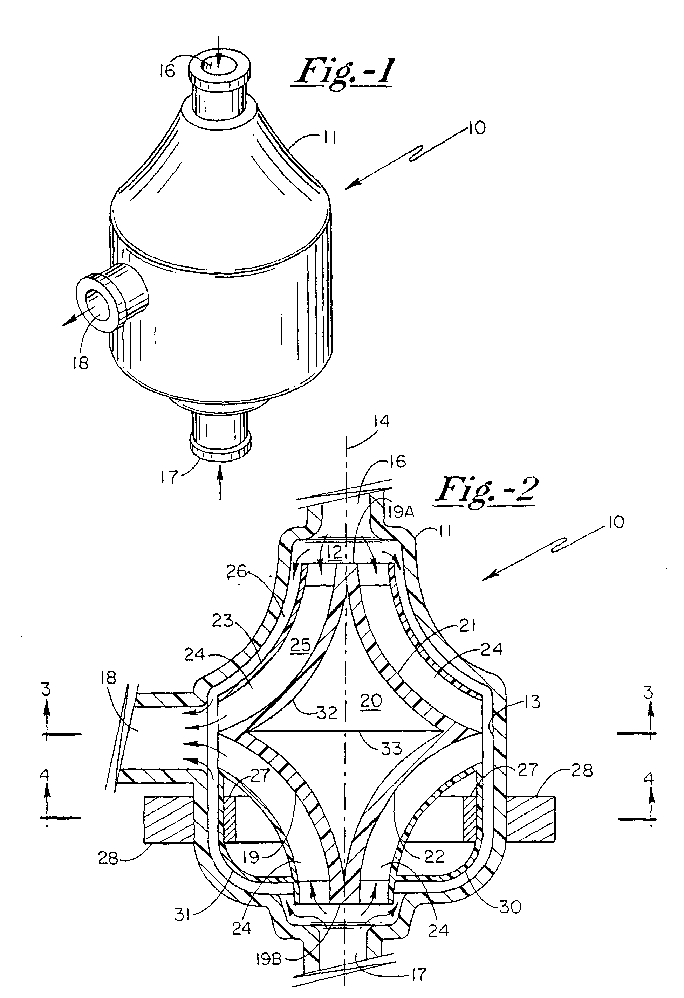

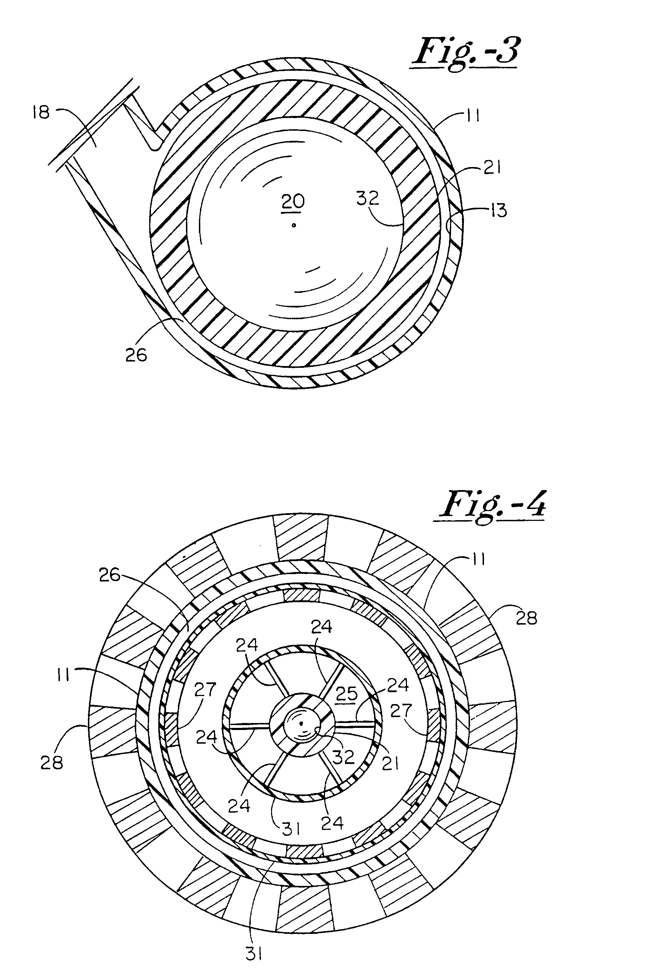

In accordance with the preferred embodiment of the present invention, and with particular attention being directed to Figures 1, 2 and 3 of the drawings, the pump generally designated 10 comprises a housing 11, the interior of which defines pumping chamber shown generally at 12. In other words, the inner periphery 13 of housing 11 is the outer periphery of the chamber 12. As is clear from the views of Figures 2 and 3, housing 11 and chamber 12 share a central axis which extends along axis 14 as set forth in Figure 2. Housing 11, and accordingly chamber 12, is provided with a pair of inlet ports as at 16 and 17, along with an outlet port as at 18. Inlet ports 16 and 17, collectively, define the inlets to the chamber, while outlet port 18 defines the outlet. The inlet ports 16 and 17 are arranged coaxially with the chamber, that is, along axis 14, with the inlet ports being arranged transverse to axis 14 and in oppositely disposed relationship to chamber 12. Outlet port 18 is arranged medially of the inlet ports, and is, as indicated, disposed generally transversely of axis 14.

With continued attention being directed to Figures 2 and 3 of the drawings, rotor generally shown at 20 is disposed within chamber 12 and has a symmetrical dual conical configuration. This configuration provides a core member 19 with dual cones converging toward opposed polar regions such as 19A and 19B, and the rotor is provided with an axis of rotation which extends between the polar regions. The base of each of the two cones forming the dual cone configuration of core 19 are coupled together and form a common center plane. Positioned between core 19 and shroud 23 are a plurality of vanes, with the opposed ends of the vanes being shown in Figure 2. These vanes are also shown in section in Figure 4. Coupled to core 19 is a shroud 23, with shroud 23 being coupled to core 19 by means of coupling rods or posts 24-24, thereby creating an additional fluid contact area for inducing flow, along with an annular flow channel as shown generally at 25. An external flow channel is also defined annularly and externally of rotor assembly 20 as at 26.

A plurality of permanent magnets are provided as at 27-27, with these magnets being arranged at radially spaced locations below or above the medial plane of rotor 20 and along the axis of rotation of the rotor, with the permanent magnets being provided at equally radially and arcuately spaced locations. Electromagnetic drive means are provided as at 28-28, with the electromagnetic drive means being, in turn, coupled to a source of electrical energy and arranged to deliver rotational driving energy to the rotor through the permanent magnets 27-27. The drive arrangement is, of course, commonly referred to as a brushless motor configuration and brushless motor drives are, of course, well known in the art. The rate of rotation of rotor 20 is conveniently controlled by means of the frequency of the field applied to electromagnetic members 28-28, with the rate of rotation being controlled by the frequency of the applied electromagnetic field, or by selective energization of the electromagnetic means 28-28. Such drives are, of course, commonly utilized and well known in the art.

Rotor 20 is further defined by walls 21 and 22 along with shroud 23, with the material of construction being either similar or identical to that employed in housing 11. A suitable biocompatible material such as polycarbonate, acrylic, or copolymers of polystyrene may be employed, or alternatively a coating may be applied to a suitable substrate in order to enhance the biocompatibility of the structure. In those instances where the device is not being employed for implantation, then, of course, other materials may be employed, provided that the blood-contacting surfaces be formed and/or coated with a non-thrombogenic material.

Rotor 20 is provided with a hollow core or void area internally of surface 32, with this area providing a means for controlling the relative density of the rotor body. Preferably, the relative density is selected by the ratio of the relative density of the rotor to that of the fluid being pumped, and in most applications, the relative density of the rotor to the fluid being pumped is between about 0.3 and 0.6, with it being understood that relative densities of between about 0.1 and 0.9 may be found useful. In the event the rotor material has a density which is lower than that of a fluid to be pumped, the voids in the core and shroud may, of course, be eliminated.

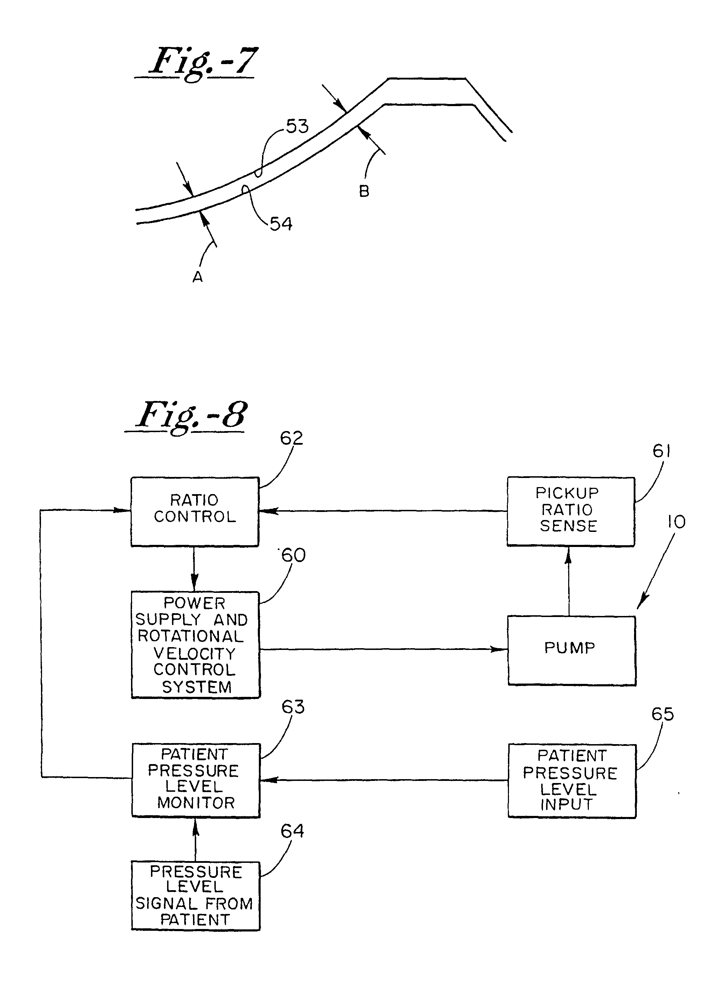

The dual conical configuration of rotor 20 and its shroud 23 provides the finished structure with an axial length along the axis of rotation as being generally equal to the axial length of the pumping chamber between the inlet ports 16 and 17. The transverse diameter of the rotor 20 is defined along a medial plane, as along medial line 33 and with the configuration of the dual converging cones providing a clearance between the surface of the shroud and the inner surface of the pumping chamber as illustrated in greater detail in Figure 7. Generally speaking, the clearance as indicated at A-A and B-B is such that the clearance is shown substantially constant from the inlet port area to the outlet port area, however this clearance may also slightly diverge or converge toward the outlet. The dimensional clearance is sufficient to provide for a flow rate which is adequate to assure laminar flow between the zone of the polar tip to the medial plane. The design of the shroud is undertaken to assist in preserving such laminar flow. With these considerations in mind, the clearance between the inner surface of the pumping chamber and the periphery of the rotor shroud preferably ranges from between about 1 millimeter up to about 7 millimeters, with a narrower range of between about 1 millimeter and 3 millimeters being generally preferred. Generally, a clearance of about 1.5 millimeters between the outer surface of the shroud 23 and the inner surface 13 of housing 11 is preferred.

With respect to the areas of the inlet and outlet ports, it is generally preferred that the combined area of the inlet ports be at least generally equal to the area of the outlet port, thereby providing more consistency in flow and pressures, and also providing for an appropriate hydrodynamic balancing of the rotor 20 within the chamber 12. In the event multiple outlet ports are employed, then and in that event, it remains preferable that the combined area of the outlet ports be generally equal to the combined area of the inlet ports.

As has been indicated, the drive means for the electromagnetic drive elements 28-28 are preferably in the form of conductor windings, and for purposes of achieving appropriate hydrodynamic balance, the windings are carefully controlled and selectively made so as to preserve the hydrodynamic balance of the rotating rotor while eliminating the need for any form of bearing.

As has been indicated, the moment of inertia of the impeller is effectively minimized by virtue of the positioning of the mass of the impeller closer to the center of gravity (or center of mass). This may be obtained by moving the mass of the impeller needed for structural integrity closer to the center, and generally as closely as possible to the rotational axis. The moment of inertia may be controllably adjusted in connection with the structure of the present invention by arranging and mounting the permanent magnets within a circular or annular zone which is as close as possible to the maximum radius of the rotor shroud, as required, while increasing the strength of the structure along its axis of rotation. This feature is illustrated in Figure 2 wherein the permanent magnets 27-27 are disposed adjacent the outer circumference of lower shroud segment 30.

Accordingly, in the configuration illustrated in Figure 2, lower shroud segment 30, while concentrically arranged relative to rotor core 19, this segment of the shroud encloses or otherwise encapsulates permanent magnets 27-27, while at the same time arranging an annular flow channel as at 31.

With respect to the fluid being pumped, it should be noted that the human blood has a viscosity of about 4 centipoises at 25° C., and this viscosity is sufficient to provide for sufficient friction between a relatively smooth rotating surface and blood so as to achieve a sufficient rotational component of motion for hydrodynamic balancing. In the shrouded rotor configuration illustrated herein, it will be appreciated that the shroud provides additional contact area, thus accommodating the utilization of relatively smooth rotating surfaces and fluid blood.

As the rotational velocity of the fluid being pumped increases, its hydrodynamic balance effect will, of course, increase correspondingly and proportionately. With a rotational velocity of approximately 1000 rpm, the hydrodynamic balancing effect substantially overwhelms the buoyant effect afforded by the relative density of the rotor within the chamber.

For start-up purposes, saline is normally preferred as the functional material, with the saline being employed for a period of time until the desired rotational velocity is achieved, and thereafter blood may be introduced as the working solution being pumped and/or transferred.

While the rotor structure illustrated is described as being relatively smooth, vanes may be employed on the structure with the vanes forming arcuately spaced passages within the rotor. In other words, the vanes may be formed as individual arcuately spaced paddles to form spaced-apart fluid passages and/or channels. A plurality of vanes are positioned between the outer surface of core 19 and the inner surface of shroud 23 as illustrated in Figures 2 and 4. Additionally, if desired, the configuration of support may be such that these components of the assembly function as vanes as well. Thus, while vanes as illustrated have rounded edges, other vane configurations such as elliptical may be employed.

The inlet and outlet diameters are preferably 7 millimeters and the relative density is preferably between 0.1 to 0.9, with a relative density of 0.5 being preferred.

For most operational purposes, an inlet pressure ranging from between about 5 millimeters of Hg (mercury) up to about 40 millimeters Hg (mercury) is considered normal and appropriate for fluid dynamics dealing with human blood. Outlet pressures of between about 40 millimeters Hg (mercury) up to about 150 or 200 millimeters Hg (mercury) may be employed. When the device of the present invention is functioning as an implantable unit, the outlet pressure will, of course, depend upon the patient's activity and circulatory requirements being indicated.

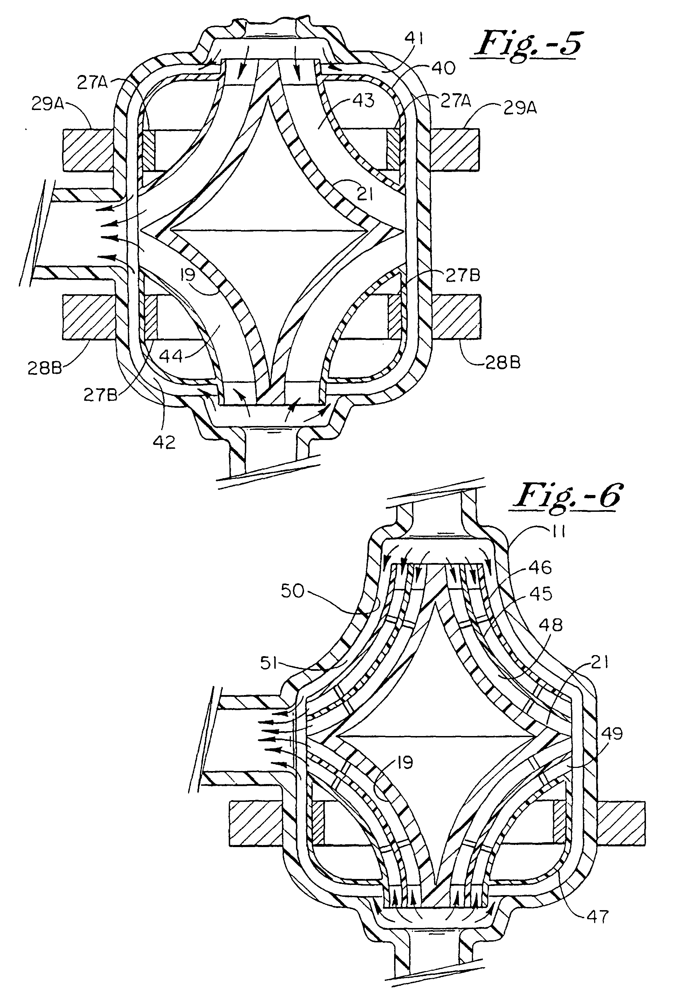

Attention is now directed to Figure 5 of the drawings wherein a modified drive and shroud configuration is illustrated. In Figure 5, for example, shroud 40 is symmetrically arranged about rotor core 19. In this connection, however, both upper and lower portions of shroud 40 are symmetrical, and provide secondary annular flow channels as at 41 and 42. Additionally, main or primary annular flow channels are provided as at 43 and 44, as shown in the drawings.

In this arrangement, however, symmetrically arranged dual drive mechanisms are provided with permanent magnet assemblies being shown at 27A and 27B respectively, and with drive magnets being shown at 28A, 28B, 29A, and 29B, respectively. With the exception of the shroud design, the other features of the configuration of Figure 5 are the same as those illustrated and described in connection with Figures 1 and 2 hereinabove.

With attention now being directed to Figure 6 of the drawings, a modified shroud configuration is illustrated, with rotor core 19 being provided with a pair of concentrically arranged shrouds as at 45 and 46 respectively. In the arrangement of Figure 6, it will be noted that inner shroud 45 is totally symmetrical about rotor core 19, while outer shroud 46 is provided with a lower segment or portion as at 47 which is similar in its configuration to shroud portion 30 as illustrated in Figure 2. In the configuration of Figure 6, multiple annular flow channels are provided between the rotor core and the inner shroud as at 48, between inner and outer shrouds as at 49, and in the outer annular zone between outer shroud 46 and the inner surface 50 of housing 11, with this outer annular channel being shown at 51. The rotor configuration with multiple shrouds as shown in Figure 6 may be modified in the manner of the structure illustrated in Figure 5 with a dual drive mechanism.

With attention now being directed to Figure 7 of the drawings, this figure, which is a fragmentary sectional view, illustrates the configuration of the clearance between the outer surface of the rotor shroud and the housing. In this view, the inner surface of the housing is illustrated at 53, with the outer surface of the shroud being illustrated at 54.

Attention is now directed to Figure 8 of the drawings wherein the pump 10 is coupled in a system which functions as a ventricular or heart-assist device. Pump 10 is powered by power supply 60 and sensors, including pickup ratio sensor 61 and ratio control 62 are employed. The patient pressure level monitor 63 provides an input to ratio control 62 with the level monitor receiving information including patient pressure level input as at 64 and pressure level signal 65. These systems are known in the art and may be employed effectively in connection with the device of the present invention.

While double shrouds have been discussed, it is possible that multiple shrouds may be employed wherein the rotor core is provided with surfaces of revolution disposed axially outwardly of the outer core surface, and with the surfaces of revolution being arranged coaxially with the axis of rotation of the rotor.

While the term "double conical configuration" has been employed throughout for the rotor core, it will be understood that other surfaces of revolution may be employed, such as those surfaces of revolution generated by a curved line such as parabola, or a straight line so as to form a cone. Thus, the term "cone" is understood to be broadly defined herein. Additionally, modified surfaces of revolution such as those illustrated in connection with the shroud of Figure 5 may be utilized. DESCRIPTION OF A SECOND OR ALTERNATE PREFERRED EMBODIMENT

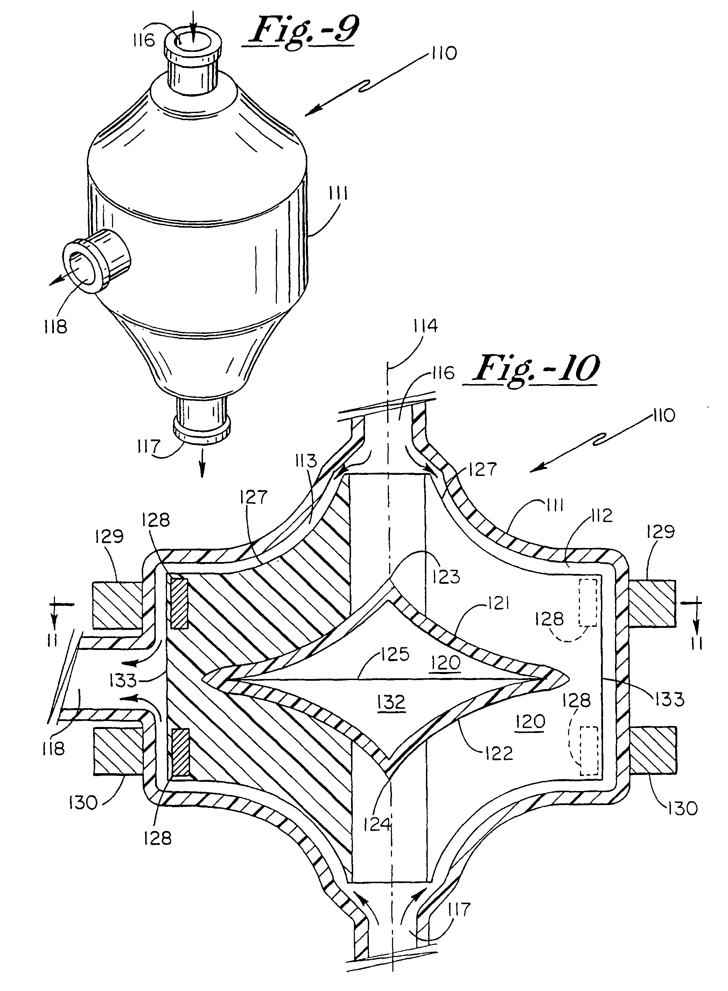

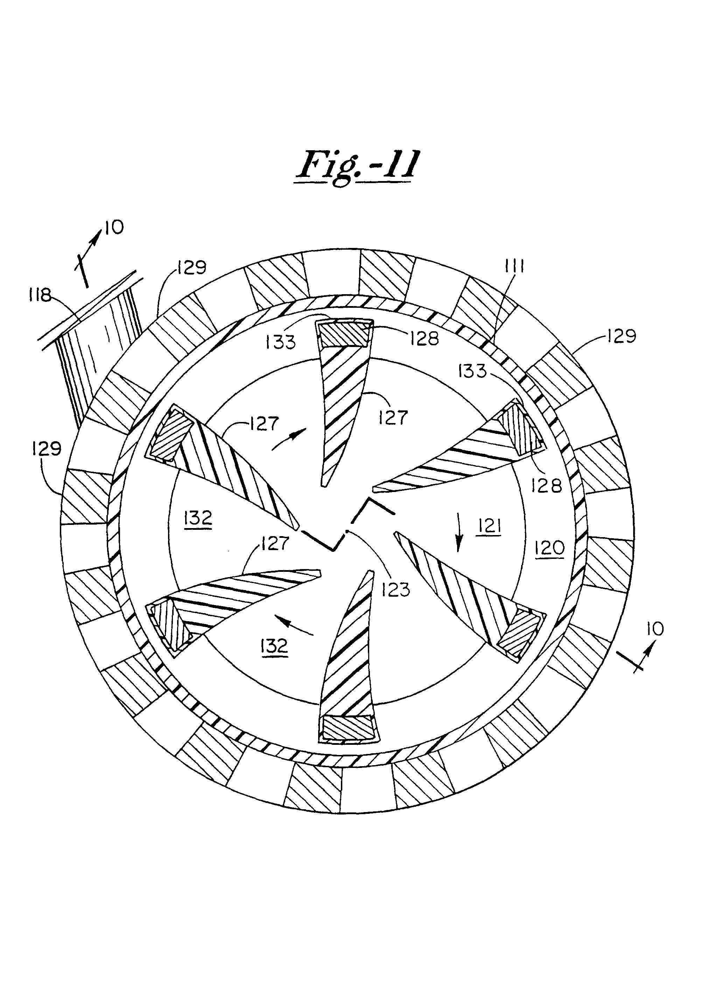

In accordance with a second preferred embodiment of the present invention, and with particular attention being directed to Figures 9, 10 and 11 of the drawings, the pump generally designated 110 comprises a housing 111, the interior of which defines pumping chamber 112. In other words, the inner periphery 113 of housing 111 is the outer periphery of the chamber 112. As is clear from the views of Figures 10 and 11, housing 111 and chamber 112 share a central axis which extends along axis 114 as set forth in Figure 10. Housing 111, and accordingly chamber 112, is provided with a pair of inlet ports as at 116 and 117, along with an outlet port as at 118. Inlet ports 116 and 117, collectively, define the inlets to the chamber, while outlet port 118 define the outlet. The inlet ports 116 and 117 are arranged coaxially with the chamber, that is, along axis 114, with the inlet ports being arranged in oppositely disposed relationship to chamber 112. Outlet port 118 is arranged medially of the inlet ports, and is, as indicated, disposed generally transversely of axis 114. It should be noted that multiple outlet ports may be utilized, and in such an event, each port is typically arranged medially of the inlet ports, consistent with the arrangement illustrated for outlet port 118.

With continued attention being directed to Figures 10 and 11 of the drawings, rotor generally designated 120 is disposed within chamber 112 and has a modified symmetrical dual conical configuration. This configuration provides dual cones 121 and 122 converging toward opposed polar regions such as 123 and 124, and the rotor is provided with an axis of rotation which extends between the polar regions 123 and 124, which is normally consistent with and coaxially with axis 114. The base of each of the two cones forming the dual cone configuration are coupled together and form a common center plane 125. The outer surfaces of dual cones 121 and 122 are provided with a plurality of radial vanes such as at 127-127. Vanes 127-127 are arranged with surface portions extending axially and radially of the outer surfaces of cone members 121 and 122, as is illustrated and shown in Figure 10. In addition, vanes 127-127 are utilized as a mounting base for a plurality of permanent magnets such as magnets 128-128. These magnets are arranged at radially spaced locations generally equally axially spaced from center plane 125 and radially outwardly of the axis of rotation of rotor 120. The permanent magnets 128-128 are provided at equally radially and arcuately spaced locations. Electromagnetic drive means are provided as at 129-129 and 130-130, with the electromagnetic drive means being, in turn, coupled to a source of electrical energy and arranged to deliver rotational driving energy to the rotor through the permanent magnets 128-128. The drive arrangement is, of course, commonly referred to as a brushless motor configuration and brushless motor drives are, of course, well known in the art. The rate of rotation of rotor 120 is conveniently controlled by means of the frequency of the field applied to electromagnetic members 129-129 and 130-130, with the rate of rotation being controlled by the frequency of the applied electromagnetic field, or by selective energization of the electromagnetic means 129-129 and 130-130. Such drives are, of course, commonly utilized and well known in the art.

The material of construction of rotor 120 is either similar or identical to that employed in housing 111. A suitable biocompatible material such as polycarbonate, acrylic, or copolymers of polystyrene may be employed, or alternatively a coating may be applied to a suitable substrate in order to enhance the biocompatibility of the structure. In those instances where the device is not being employed for implantation, then, of course, other materials may be employed, provided that the blood-contacting surfaces be formed and/or coated with a non-thrombogenic material.

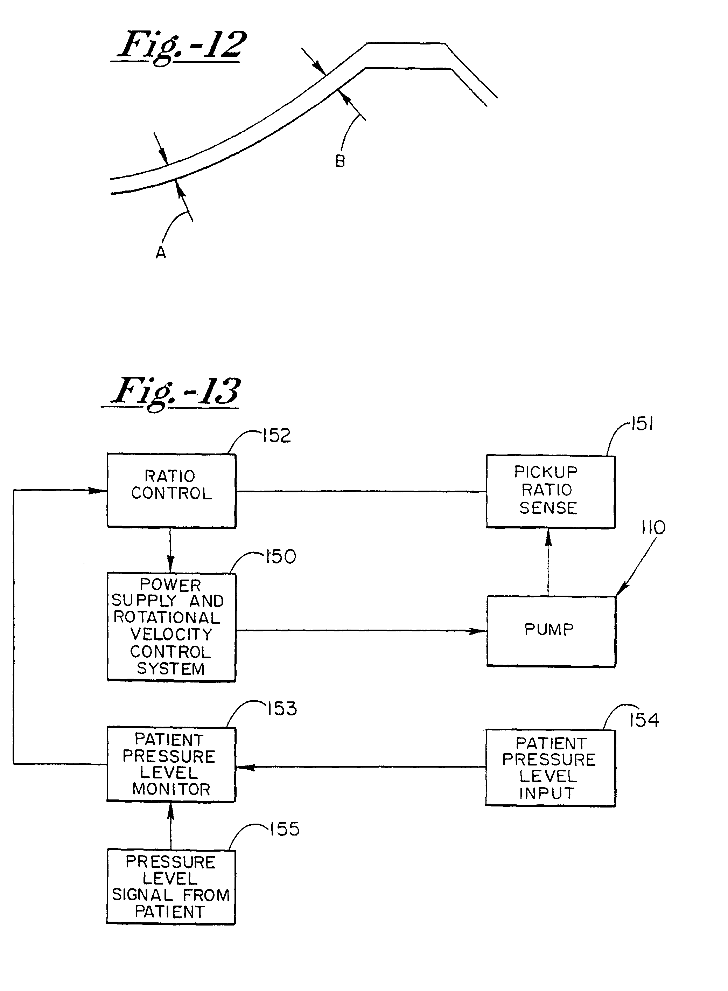

Rotor 120 is provided with a hollow core or void chamber area as shown generally at 132, with this volume or area providing a means for controlling the relative density of the rotor body. Preferably, the relative density is selected by the ratio of the relative density of the rotor to that of the fluid being pumped, and in most applications, the relative density of the rotor to the fluid being pumped is between about 0.3 and 0.6, with it being understood that relative densities of between about 0.1 and 0.9 may be found useful. Also, the dual conical configuration of rotor 120 provides the finished structure with an axial length along the axis of rotation as being generally equal to the axial length of the pumping chamber between the inlet ports 116 and 117. The transverse diameter of the rotor 120 is defined along a medial plane, as along medial line 125 and with the configuration of the dual converging cones and the radial vanes providing a clearance between the surface of the vanes of the rotor and the inner surface of the pumping chamber as illustrated in greater detail in Figure 12. Generally speaking, the clearance as indicated at A-A and B-B is such that the clearance remains constant from the inlet port area to the outlet port area, however this clearance may be slightly divergent or slightly convergent toward the outlet. The spacing between the periphery of the vanes as at 132 and the outer surface of dual cones 121 and 122 is preferably approximately constant, and also generally parallel to the inner surface 113 of housing 111. With the volume available for transfer of the fluid being pumped, the meridional velocity of the pumped fluid remains generally constant during its passage through the pump as it moves along its translational and rotational paths and/or vectors. With these considerations in mind, the clearance between the inner surface of the pumping chamber and the outer edges of the radial vanes 127-127 or rotor 120 preferably ranges from between about 1 millimeter up to about 7 millimeters, with a narrower range of between about 1 millimeter and 3 millimeters being generally preferred. Generally, a clearance of about 1.5 millimeters is preferred, with this spacing being dimensionally illustrated at the zone between opposed arrows A-A and B-B of Figure 12.

With respect to the areas of the inlet and outlet ports, it is generally preferred that the combined area of the inlet ports be generally equal to the combined areas of the outlet port or ports, thereby providing more consistency in flow and pressures, and also providing for an appropriate hydrodynamic balancing of the rotor 120 within the chamber 112.

The permanent magnets 128-128 are encapsulated within the radial vanes 127-127. As illustrated in Figure 10, the physical position of the encapsulated permanent magnets 128-128 is generally closely adjacent the outer radial edge surface of vanes 127-127, with the radially outward edge surface being illustrated at 133.

As has been indicated, the drive means for the electromagnetic drive elements 129-129 and 130-130 is preferably in the form of conductor windings, and for purposes of achieving appropriate hydrodynamic balance, the windings are carefully controlled and selectively made so as to preserve the hydrodynamic balance of the rotating rotor while eliminating the need for any form of bearing.

The moment of inertia of the rotor or impeller is effectively minimized by virtue of the positioning of the mass of the impeller closer to the center of gravity or center of mass. This may be obtained by moving the mass of the impeller needed for structural integrity closer to the center, and generally as closely as possible to the rotational axis. The moment of inertia may be controllably adjusted in connection with the structure of the present invention by arranging and mounting the permanent magnets within a circular or annular zone which is at the maximum radius of the rotor inner impeller, as required, while at the same time increasing the strength of the structure along its axis of rotation.

With respect to the fluid being pumped, it should be noted that the human blood has a viscosity of about 4 centipoises at 25° C., and this viscosity is sufficient to adequately provide for sufficient friction between the vanes and rotor surface against the blood so as to achieve a sufficient rotational component of motion for hydrodynamic balancing. As the rotational velocity of the fluid being pumped increases, its hydrodynamic balance effect will, of course, increase correspondingly and proportionately. With a rotational velocity of approximately 1000 rpm, the hydrodynamic balancing effect substantially overwhelms the buoyant effect afforded by the relative density of the rotor within the chamber.

For start-up purposes, saline is normally preferred as the functional material, with the saline being employed for a period of time until the desired rotational velocity is achieved, and thereafter blood may be introduced as the working solution being pumped and/or transferred.

The inlet and outlet diameters are preferably 7 millimeters and the relative density is preferably between 0.1 to 0.9, with a relative density of 0.5 being preferred.

For most operational purposes, an inlet pressure ranging from between about 5 millimeters of Hg (mercury) up to about 40 millimeters Hg (mercury) is considered normal and appropriate for fluid dynamics dealing with human blood. Outlet pressures of between about 40 millimeters Hg (mercury) up to about 150 or 200 millimeters Hg (mercury) may be employed. When the device of the present invention is functioning as an implantable unit, the outlet pressure will, of course, depend upon the patient's activity and circulatory requirements being indicated.

The pump device of the present invention may be utilized as a patient-assist unit, with the pump being employed as a device with the outlet or outlets coupled to the aorta. In an alternative construction, the outlet may be coupled to the pulmonary artery. As indicated, the device of the present invention has application as a transfer pump as well, and may be employed, therefore, in surgical procedures which involve temporarily removing and/or temporarily disabling the heart function.

Attention is now directed to Figure 13 of the drawings wherein the pump 110 is coupled in a system which functions as a ventricular or heart-assist device. Pump 110 is powered by power supply 150 and sensors, including pickup ratio sensor 151 and ratio control 152 are employed. The patient pressure level monitor 153 provides an input to ratio control 152 with the level monitor receiving information including patient pressure level input as at 154 and pressure level signal 155. These systems are known in the art and may be employed effectively in connection with the device of the present invention.

Again, as stated in connection with the earlier described preferred embodiment, while the term "double conical configuration" has been employed throughout, it will be understood that other surfaces of revolution may be employed, such as those surfaces of revolution generated by a curved line such as parabola, or a straight line so as to form a cone. Thus, the term "cone" is understood to be broadly defined herein.

### DESCRIPTION OF A THIRD OR ADDITIONAL ALTERNATE PREFERRED EMBODIMENT

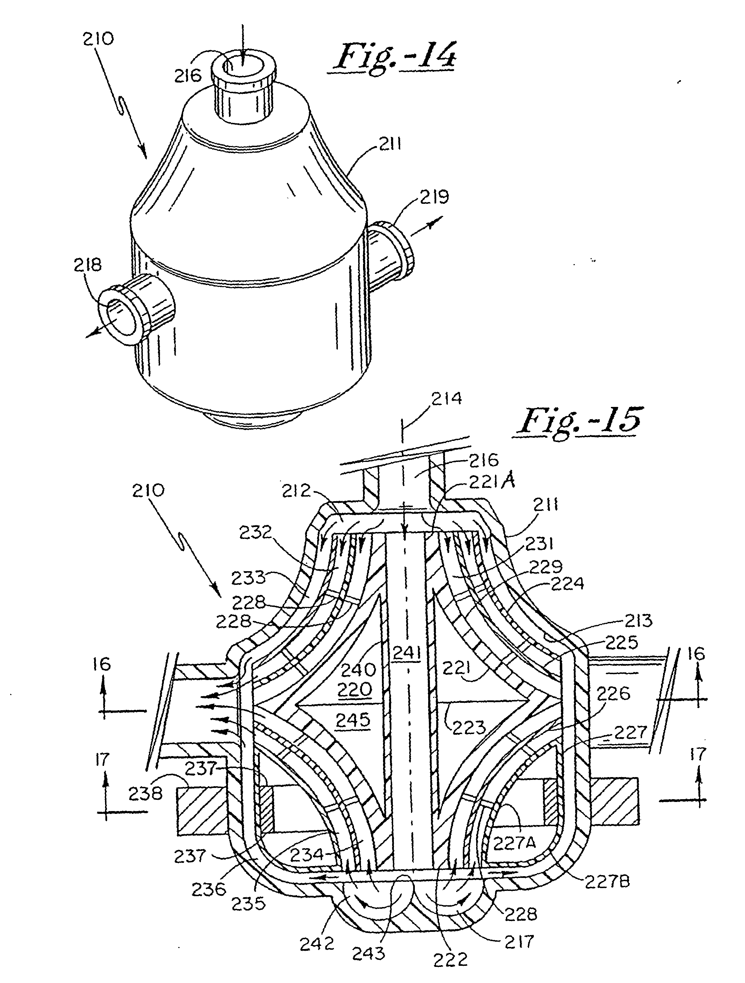

In accordance with a third or additional alternate preferred embodiment of the present invention, and with particular attention being directed to Figures 14, 15 and 16 of the drawings, the pump generally designated 210 comprises a housing 211, the interior of which defines pumping chamber shown generally at 212. In other words, the inner periphery 213 of housing 211 is the outer periphery of the chamber 212. As is clear from the views of Figures 15, 16, and 17, housing 211 and chamber 212 share a central axis which extends along axis 214 as set forth in Figure 15. Housing 211, and accordingly chamber 212, is provided with a primary inlet port as at 216, along with outlet ports as at 218 and 219. Inlet port 216 defines the inlet to the chamber, while outlet ports 218 and 219 collectively define the outlets. The external inlet port 216 is arranged coaxially with the chamber, that is, along axis 214, with the external inlet port being arranged in oppositely disposed relationship to secondary or internal inlet 217 adjacent apex 243 of deflector tip. Outlet ports 218 and 219 are arranged medially of the primary and secondary inlet ports, and are, as indicated, disposed generally transversely of axis 214.

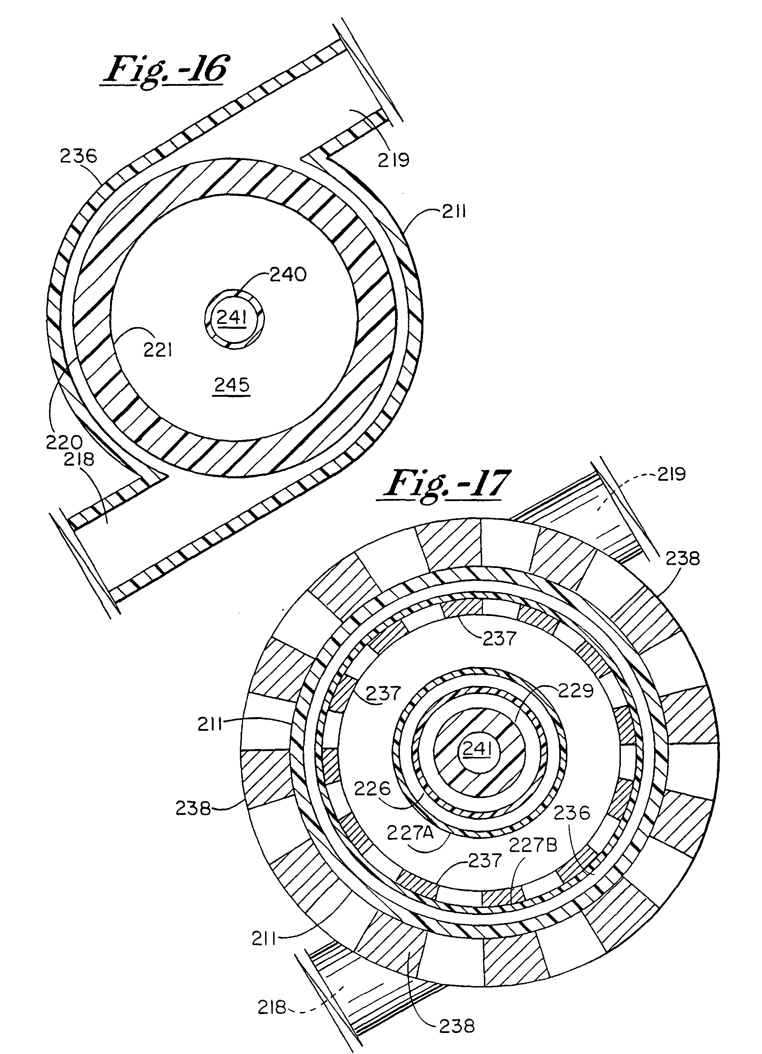

With continued attention being directed to Figures 15 and 16 of the drawings, rotor 220 is disposed within chamber 212 and has a core component 221 with a symmetrical dual conical configuration. This configuration provides dual cones of arcuate configuration converging toward opposed polar regions such as 221A and 222, with the rotor being provided with an axis of rotation which extends between the polar regions 227Å and 222, and generally along with and coincidental with axis 214. The base of each of the two cones forming the dual cone configuration are coupled together and form a common center plane as at 223. The rotor core 221 is also provided with a pair of conically configured shrouds, such as at 224, 225, 226 and 227. Shrouds 224, 225 and 226 are each generally conical in configuration with the cones being formed as the surface of revolution of an arcuate segment, the arcuate segment having a radius which substantially matches that of the dual cones. Additionally, shroud 227 has an inner arcuate segment as at 227A along with an outer segment as at 227B. Segments 227A and 227B are joined together at their ends so as to form an enclosed surface of revolution defined generally by arcuate segments 227A and 227B, with the resultant being a member having a modified toroidal configuration.

The individual shrouds are coupled to core component 21 by posts or spoked discs 228-228. Discs 228-228 provide a link between the outer surface 229 of core component 221, and thus provide the mechanical stability and rigidity for the overall assembly. The configuration of the core component 221 together with shrouds 224, 225, 226 and 227 define annular flow channels as at 231, 232, and 233 on the upper portion of the assembly along with similar annularly configured channels 234, 235, and 236 adjacent the lower portion of the assembly. Within the confines of modified toroidal member 227 are a series of permanent magnets such as magnets 237-237. These magnets are arranged at radially spaced locations generally medially along the axis of rotation of rotor 220, with the permanent magnets being provided at equally radially and arcuately spaced locations. Electromagnetic drive means are provided as at 238-238, with the electromagnetic drive means being, in turn, coupled to a source of electrical energy and arranged to deliver rotational driving energy to the rotor through the permanent magnets 237-237. The drive arrangement is, of course, commonly referred to as a brushless motor configuration and brushless motor drives are, of course, well known in the art. The rate of rotation of rotor 220 is conveniently controlled by means of the frequency of the field applied to electromagnetic members 238-238, with the rate of rotation being controlled by the frequency of the applied electromagnetic field, or by selective energization of the electromagnetic means 238-238. Such drives are, of course, commonly utilized and well known in the art.

Rotor 220 is defined by outer surface or wall 229, with the material of construction being either similar or identical to that employed in housing 211. A suitable biocompatible material such as polycarbonate, acrylic, or copolymers of polystyrene may be employed, or alternatively a coating may be applied to a suitable substrate in order to enhance the biocompatibility of the structure. In those instances where the device is not being employed for implantation, then, of course, other materials may be employed, provided that the blood-contacting surfaces be formed and/or coated with a non-thrombogenic material. If the rotor material has a lower density than a pumped fluid, the voids and/or cavities formed in the core and shroud may be eliminated.

Rotor 220 is provided with a tubular core 240 which defines an axial bore generally designated 241. Bore 241 accepts fluid from inlet 216 and transmits the fluid directly to secondary inlet zone or chamber 242, for ultimate transfer and flow through annular channels 234, 235 and 236. As indicated, each of these channels leads to outlet 218, thereby providing continuity of flow. An apex point is provided as at 243 in order to provide for smooth flow, preferably laminar flow, through the pump assembly.

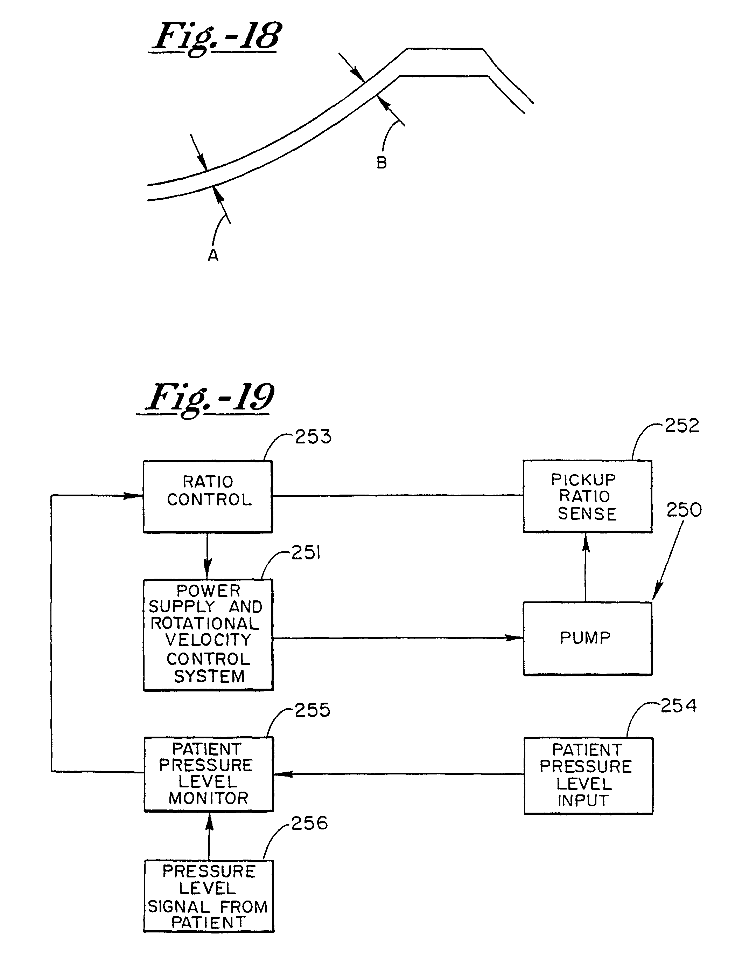

In addition, a hollow core or void area is provided within rotor core 221, with this chamber being shown generally at 245. This chamber area provides a volume of a size or magnitude which in turn defines a means for controlling the relative density of the rotor body. Additional buoyancy is provided by the modified toroidal configuration of member 227, thereby providing an overall relative density for the rotor assembly. Preferably, the relative density is selected by the ratio of the relative density of the rotor or rotor assembly to that of the fluid being pumped, and in most applications, the relative density of the rotor to the fluid being pumped is between about 0.3 and 0.6, with it being understood that relative densities of between about 0.1 and 0.9 may be found useful. Also, the dual conical configuration of the core component 221 of rotor 220 together with shrouds provides the finished structure with an axial length along the axis of rotation as being generally equal to the axial length of the pumping chamber between the primary inlet port 216 and secondary inlet port area 242. The transverse diameter of the rotor 220 and its shrouds is defined along a medial plane, as along medial line 223 and with the configuration of the dual converging cones providing a clearance between the surface of the rotor and the inner surface of the pumping chamber as illustrated in greater detail in Figure 18. Generally speaking, the clearance as indicated at A-A and B-B is such that the clearance remains constant from the primary and secondary inlet ports to the outlet ports. The combined area of the individual flow channels is such that the rate of motion for the fluid being pumped as it moves along its transitional and rotational motions and/or vectors is substantially constant. With these considerations in mind, the clearance between the inner surface of the pumping chamber and the periphery of the rotor preferably ranges from between about 1 millimeter up to about 7 millimeters, with a narrower range of between about 1 millimeter and 3 millimeters being generally preferred for blood. Generally, a clearance of about 1.5 millimeters is preferred.

With respect to the areas of the inlet and outlet ports, it is generally preferred that the combined area of the primary inlet port 216 is generally equal to the combined areas of the outlet ports 218 and 219, thereby providing more consistency in flow and pressures, and also providing for an appropriate hydrodynamic balancing of the rotor 220 within the chamber 212.

As has been indicated, the drive means for the electromagnetic drive elements 238-238 is preferably in the form of conductor windings, and for purposes of achieving appropriate hydrodynamic balance, the windings are carefully controlled and selectively made so as to preserve the hydrodynamic balance of the rotating rotor while eliminating the need for any form of bearing.

As has been indicated, the moment of inertia of the impeller is effectively minimized by virtue of the positioning of the mass of the impeller closer to the center of gravity or center of mass. This may be obtained by moving the mass of the impeller needed for structural integrity closer to the center, and generally as closely as possible to the rotational axis. The moment of inertia may be controllably adjusted in connection with the structure of the present invention by arranging and mounting the permanent magnets within a circular or annular zone which is at the maximum radius of the rotor inner impeller, as required, while increasing the strength of the structure along its axis of rotation. As is indicated in Figure 15, for example, permanent magnets 237-237 are positioned within the confines of modified toroidal member 227, thereby effectively isolating the permanent magnets from contact with the fluid being pumped.

With respect to the fluid being pumped, it should be noted that the human blood has a viscosity of about 4 centipoises at 25° C., and this viscosity is sufficient to provide for sufficient friction between relatively smooth rotor and shroud surfaces and blood so as to achieve a sufficient rotational component of motion for hydrodynamic balancing. As the rotational velocity of the fluid being pumped increases, its hydrodynamic balance effect will, of course, increase correspondingly and proportionately. With a rotational velocity of approximately 1000 rpm, the hydrodynamic balancing effect substantially overwhelms the buoyant effect afforded by the relative density of the rotor within the chamber.

For start-up purposes, saline is normally preferred as the functional material, with the saline being employed for a period of time until the desired rotational velocity is achieved, and thereafter blood may be introduced as the working solution being pumped and/or transferred.

While the rotor structure illustrated is described as being relatively smooth, vanes may be employed on the structure with the vanes forming arcuately spaced passages within the rotor. In other words, vanes, if employed, may be formed as individual arcuately spaced paddles to form spaced-apart fluid passages and/or channels.

The inlet and outlet diameters are preferably 7 millimeters and the relative density is preferably between 0.1 to 0.9, with a relative density of 0.5 being preferred.

For most operational purposes, an inlet pressure ranging from between about 5 millimeters of Hg (mercury) up to about 40 millimeters Hg (mercury) is considered normal and appropriate for fluid dynamics dealing with human blood. Outlet pressures of between about 40 millimeters Hg (mercury) up to about 150 or 200 millimeters Hg (mercury) may be employed. When the device of the present invention is functioning as an implantable unit, the outlet pressure will, of course, depend upon the patient's activity and circulatory requirements being indicated.

Attention is now directed to Figure 19 of the drawings wherein a system is illustrated for utilization of the pump device of the present invention as a patient-assist unit. In the drawing of Figure 19, the pump 250 may be employed as a device with the outlets coupled to the aorta. In an alternative construction, the outlet may be coupled to the pulmonary artery. As indicated, the device of the present invention has application as a transfer pump as well, and may be employed, therefore, in surgical procedures which involve temporarily removing and/or temporarily disabling the heart function.

The pump 250 is coupled in a system which functions as a ventricular or heart-assist device. Pump 250 is powered by power supply 251 and sensors, including pickup ratio sensor 252 and ratio control 253 are employed. The patient pressure level monitor 255 provides an input to ratio control 253 with the level monitor 255 receiving information, including patient pressure level input as at 254 and pressure level signal 256. These systems are known in the art and may be employed effectively in connection with the device of the present invention.

While double cones have been discussed, it is possible that multiple cones may be employed in lieu of vanes, wherein the rotor is provided with surfaces of revolution disposed axially outwardly of the rotor, and with the surfaces of revolution being arranged coaxially with the axis of rotation of the rotor.

While the term "double conical configuration" has been employed throughout, it will be understood that other surfaces of revolution may be employed, such as those surfaces of revolution generated by a curved line such as parabola, hyperbola, or a straight line so as to form a cone. Thus, the term "cone" is understood to be broadly defined herein.

## Claims

1. A pump (210) for transferring fluids and comprising a pumping chamber (212) within a housing (211) having an inner periphery (213), an outer periphery and a central axis (214), primary and secondary fluid inlet ports (216, 217) arranged in oppositely disposed relationship on said housing (211) and coaxially with said pumping chamber (212), an outlet port means (218, 219) arranged transversely and generally medially of said primary and secondary inlet ports (216, 217), a rotor (220) disposed within said pumping chamber (212) and having a core with a dual conical configuration converging toward opposed polar regions (221A, 222) and having an axis of rotation extending between said polar regions (221A, 222) and arranged coaxially with the axis (214) of said housing (211) during operational rotation of said rotor (220), magnetic driven means (237) arranged on said rotor (220) at radially spaced locations generally from said axis of rotation, electromagnetic drive means (238) coupled to a source of energy and arranged to deliver rotational driving energy to said rotor (220) through said magnetic driven means (237); said rotor (220) comprising:
(a) a core (221) with at least one shroud (225) coupled to the outer surface of said core (221) having a conical configuration with a core axial length and with the core axial length defining the axial length of said pumping chamber (212), and with said core (221) being disposed between said primary and secondary inlet ports (216, 217); and with the diameter of said core (221) and shroud (225) transverse to said axis of rotation defining a medial plane (223) and being selected to provide a clearance between the outer surface of said shroud (225) and the inner surface (213) of said housing (211) and with the magnitude of the clearance between the said inner surface (213) of said housing (211) and the outer periphery of said shroud (225) from said primary and secondary inlet ports (216, 217) to said outlet port (218, 219) being approximately constant **characterised in that** said core (221) having a relative density substantially less than that of the fluid being pumped and within a range of between 10 percent and 90 percent of the density of the fluid being pumped, the arrangement being such that the sole support for the rotor (220) is the hydrodynamic forces created in the fluid being pumped, wherein said housing (211) and said pumping chamber (212) are free of rotor supporting members and bearings.

2. The pump (210) as in Claim 1 wherein the clearance between the inner surface (213) of said housing (211) and the outer periphery of said shroud (225) is from between about 1 millimeter and 7 millimeters.

3. The pump (210) of Claim 1 having plural outlet ports (218, 219), with the sum total of the cross-sectional area of said outlet ports (218, 219) being substantially equal to the cross-sectional area of said inlet ports (216, 217).

4. The pump (210) of Claim 3 where the said plurality of outlet ports (218, 219) are generally equally arcuately spaced, one from another.

5. The pump (210) of Claim 2 wherein said clearance between the inner surface (213) of said housing (211) and the outer surface of said shroud (225) is adequate to provide a flow channel (233) for blood, and is such that the velocity of fluid being pumped remains substantially constant between said primary and secondary inlet ports (216, 217) and said outlet ports (218, 219) relative to the surface (213) of said housing (211).

6. The pump (210) of Claim 1 wherein the rate of rotation of said rotor (220) is controllably variable.

7. The pump (210) of Claim 1 being particularly **characterized in that** means (252) are provided for sensing the rotational velocity of said rotor (220).

8. The pump (210) of Claim 1 being particularly **characterized in that** the driving forces for said rotor are coupled to said magnetic driven means (237), which are axially spaced from the center of mass of the rotor (220). (220).

9. The pump (210) of Claim 1 being particularly **characterized in that** the fluid flows from inlets (216, 217) adjacent the polar tips (221 A, 222) of the cones to outlets (218, 219) adjacent the medial plane (223).

10. The pump (210) as defined in Claim 1 wherein the drive means includes permanent magnets (237) arranged within the rotor (220) along radial points adjacent the outer circumference of the rotor.

11. The pump (210) as defined in Claim I wherein the drive means includes permanent magnets (237) disposed in a circular array, and wherein the cuter perimeter of the magnets forming the is spaced from the mid-point (223) thereof, and with the structural of the rotor (220) being disposed adjacent the rotational axis (214), thereby reducing the moment of inertia of said rotor.

## Patentansprüche

1. Pumpe (210) zum Transportieren von Flufden mit einer Pumpkammer (212) innerhalb eines Gehäuses (211), das einen Innenumfang (213), einen Außenumfang und eine Mittelachse (214) aufweist, mit primären und sekundären Fiuideinlassanschiüssen (216, 217), die in einander gegenüberliegender Relativbeziehung an dem Gehäuse (211) und koaxial zu der Pumpkammer (212) angeordnet sind, mit einem Auslassanschlussmittel (218, 219), das quer und mitten zwischen den primären und sekundären Einlassanschlüssen (216, 217) angeordnet ist, mit einem Rotor (220), der innerhalb der Pumpkammer (212) angeordnet ist und einen Kein mit einer doppeltkonischen Konfiguration aufweist, die zu einander gegenüberliegenden Polbereichen (221A, 222) konvergiert und eine Drehachse aufweist, welche sich zwischen den Poiberelchen (221A. 222) erstreckt und während der Betriebsdrehung des Rotors (220) koaxial zu der Achse (214) des Gehäuses (211)angeordnet ist, mit magnetischen Antriebsmitteln (231), die an dem Rotor (220) an allgemein von der Drehachse radial beanstandeten Orten angeordnet sind, und mit elektromagnetischen Antriebsmitteln (238), die an eine Energiequelle angeschlossen und angeordnet sind, um durch die magnetischen Antriebmittel (237) Drehantriebsenergie auf den Rotor (220) zu übertragen; wobei der Rotor (220) aufweist:
(a) einen Kern (221) mit mindestens einer Abdeckung (225), die an die Außenoberfläche des Kerns (221), welcher eine konische Konfiguration mit einer axialen Länge des Kerns aufweist, wobei die axiale Länge des Kerns die axiale Länge der Pumpkammer (212) definiert und wobei der Kern (221) zwischen den primären und sekundären Einlassanschlüssen (216, 217) angeordnet ist, und wobei der Durchmesser das Kerns (221) und der Abdeckung (225) quer zu der Drehachse eine Zwischenebene (223) definiert und gewählt ist, um einen Freiraum zwischen der äußeren Oberfläche der Abdeckung (225) und der Innenobsrfläche (213) des Gehäuses (211) bereitzustellen, und wobei die Größe des Freiraums zwischen der Innenoberfläche (213) des Gehäuses (211) und dem Außenumfang der Abdeckung (225) von den primären und sekundären Einlassanschlüssen (216, 217) zu dem Auslassanschluss (218, 219) ungefähr konstant ist, **dadurch gekennzeichnet, dass** der Kern (221) eine relative Dichte aufwelst, die wesentlich geringer als diejenige des gepumpten Fluids ist und in einem Bereich zwischen 10 und 90 % der Dichte des gepumpten Fluids liegt, wobei die Anordnung so ist, dass die einzige Abstutzung für den Rotor (220) die hydrodynamischen Kräfte sind, die in dem gepumpten Fluid erzeugt werden, wobei das Gehäuse (211) und die Pumpkammer (212) frei von rotorabstlitzenden Elementen und Lagern sind.

2. Pumpe (210) nach Anspruch 1, wobei der Freiraum zwischen der Innenoberfläche (213) des Gehäuses (211) und dem Außenumfang der Abdeckung (225) zwischen etwa 1 mm und 7 mm beträgt.

3. Pumpe (210) nach Anspruch 1 mit einer Mehrzahl von Auslassanschlüssen (218, 219), wobei die Gesamtsumme der Querschnittsfläche der Auslassanschlüsse(218, 219) Im Wesentlichen gleich der Querschnittsfläche der Einlassanschlüsse (219, 217) ist.

4. Pumpe (210) nach Anspruch 3, wobei die Mehrzahl der Auslassanschlüsse (218, 219) allgemein gleiche Bogenabstände untereinander aufweisen.

5. Pumpe (210) nach Anspruch 2, wobei der Freiraum zwischen der Innenoberfläche (213) des Gehäuses (211) und der Außenoberfläche der Abdeckung (225) angemessen ist, um einen Strömungskanal (233) für Blut bereitzustellen und derart ausgebildet ist, dass die Geschwindigkeit des gepumpten Fluids retativ zu der Oberfläche (213) des Gehäuses (211) zwischen den primären und sekundären Einlassanschlüssen (218, 217) und den Auslassanschlüssen (218, 219) im Wesentlichen konstant bleibt.

6. Pumpe (210) nach Anspruch 1, wobei die Drehzahl des Rotors (220) kontrolliert veränderbar ist.

7. Pumpe (210) nach Anspruch 1, die insbesondere **dadurch gekennzeichnet ist, dass** Mittel (252) zum Erfassen der Drehgeschwindlgkeit des Rotors (220) vorgesehen sind.

8. Pumpe (210) nach Anspruch 1, die insbesondere **dadurch gekennzeichnet ist, dass** die Antriebskräfte für den Rotor an die magnetischen Antriebsmittel (237) angeschlossen werden, die von dem Schwerpunkt des Rotors (220) axial beabstandet sind.

9. Pumpe (210) nach Anspruch 1, die insbesondere **dadurch gekennzeichnet ist, dass** das Fluid von den Einlässen (216, 217) in der Nähe der Polspitzen (221A, 222) der Konen zu den Auslässen (218,219) in der Nähe der Mittelebene (223) strömt

10. Pumpe (210) wie in Anspruch 1 definiert, wobei die Antriebsmittel Permanentmagnete (237) umfassen, die Innerhalb des Rotors (220) entlang radialen Punkten angeordnet sind, die In der Nähe des Außenumfangs des Rotors liegen.

11. Pumpe (210) wie in Anspruch 1 definiert, wobei die Antriebsmittel Permanentmagnete (237) umfassen, die in einem kreisförmigen Feld angeordnet sind, und wobei der Außenumfang der Magnete, die das Feld bilden, von dem Mittelpunkt (223) desselben beabstandet ist und wobei die strukturelle Masse des Rotors (220) in der Nähe der Drehachse (214) angeordnet ist, wodurch das Trägheitsmoment des Rotors reduziert ist.

## Revendications

1. Pompe (210) destinée au transfert de fluides et comprenant une chambre de pompage (212) située à l'intérieur d'un carter (211) comprenant une périphérie interne (213), une périphérie externe et un axe central (214), des orifices primaire et secondaire d'admission de fluide (216, 217) aménagés dans une relation disposée en opposition sur ledit carter (211) et de manière coaxiale à ladite chambre de pompage (212), des moyens d'orifice de sortie (218, 219) aménagés transversalement et généralement à mi-distance entre lesdits orifices primaire et secondaire d'admission (216, 217), un rotor (220) disposé à l'intérieur de ladite chambre de pompage (212) et comprenant un noyau ayant une double configuration conique convergeant vers des zones polaires opposées (221A, 222) et ayant un axe de rotation qui s'étend entre lesdites zones polaires (221A, 222) et aménagé de manière coaxiale à l'axe (214) dudit carter (211) au cours d'un fonctionnement en rotation dudit rotor (220), des moyens magnétiques menés (237) aménagés sur ledit rotor (220) à des emplacements espacés dans le sens généralement radial dudit axe de rotation, des moyens d'entraînement électromagnétiques (238) couplés à une source d'énergie et aménagés de manière à fournir une énergie d'entraînement en rotation audit rotor (220) par l'intermédiaire desdits moyens magnétiques menés (237) ; ledit rotor (220) comprenant :
(a) un noyau (221) comportant au moins une enveloppe (225) couplée à la surface externe dudit noyau (221) ayant une configuration conique avec une longueur axiale de noyau et la longueur axiale de noyau définissant la longueur axiale de ladite chambre de pompage (212), et ledit noyau (221) étant disposé entre lesdits orifices primaire et secondaire d'admission (216, 217) ; et le diamètre dudit noyau (221) et de ladite enveloppe (225) transversal audit axe de rotation définissant un plan médial (223) et étant sélectionné de manière à fournir un espace entre la surface externe de ladite enveloppe (225) et la surface interne (213) dudit carter (211), et l'amplitude de l'espace entre ladite surface interne (213) dudit carter (211) et la périphérie externe de ladite enveloppe (225) à partir desdits orifices primaire et secondaire d'admission (216, 217) et ledit orifice de sortie (218, 219) étant approximativement constante, **caractérisé en ce que** ledit noyau (221) a une densité relative essentiellement plus faible que celle du fluide devant être pompé et se situe dans une plage d'environ 10 % à 90 % de la densité du fluide devant être pompé, l'aménagement étant tel que le seul support du rotor (220) est la force hydrodynamique créée dans le fluide devant être pompé, dans laquelle ledit carter (211) et ladite chambre de pompage (212) ne comportent ni paliers ni éléments de support de rotor.

2. Pompe (210) selon la revendication 1, dans laquelle ledit espace entre la surface interne (213) dudit carter (211) et la périphérie externe de ladite enveloppe (225) se situe environ entre 1 millimètre et 7 millimètres.

3. Pompe (210) selon la revendication 1 comprenant une pluralité d'orifices de sortie (218, 219), la somme totale des surfaces en coupe transversale desdits orifices de sortie (218, 219) étant essentiellement égale à la surface en coupe transversale desdits orifices d'admission (216, 217).

4. Pompe (210) selon la revendication 3, dans laquelle ladite pluralité des orifices de sortie (218, 219) sont généralement espacés les uns des autres sur des arcs de cercle égaux.

5. Pompe (210) selon la revendication 2, dans laquelle ledit espace entre la surface interne (213) dudit carter (211) et la surface externe de ladite enveloppe (225) est approprié pour procurer un canal de débit (233) à du sang, et est tel que la vitesse du fluide devant être pompé reste essentiellement constante entre lesdits orifices primaire et secondaire d'admission (216, 217) et lesdits orifices de sortie (218, 219) par rapport à la surface (213) dudit carter (211).

6. Pompe (210) selon la revendication 1, dans laquelle la vitesse de rotation dudit rotor (220) peut être commandée de manière variable.

7. Pompe (210) selon la revendication 1, **caractérisée** en particulier en ce qu'il est fourni des moyens (252) destinés à détecter la vitesse de rotation dudit rotor (220).

8. Pompe (210) selon la revendication 1, **caractérisée** en particulier en ce que les forces d'entraînement destinées audit rotor sont couplées aux dits moyens magnétiques menés (237), qui sont séparés du centre de la masse du rotor (220) dans le sens axial.

9. Pompe (210) selon la revendication 1, **caractérisée** en particulier en ce que le fluide s'écoule à partir des admissions (216, 217) situées à proximité des extrémités polaires (221A, 222) des cônes jusqu'aux sorties (218, 219) situées à proximité du plan médial (223).

10. Pompe (210) selon la revendication 1, dans laquelle les moyens d'entraînement comprennent des aimants permanents (237) aménagés à l'intérieur du rotor (220) le long de points radiaux situés à proximité de la circonférence externe du rotor.

11. Pompe (210) selon la revendication 1, dans laquelle les moyens d'entraînement comprennent des aimants permanents (237) disposés en réseau circulaire, et dans laquelle le périmètre externe des aimants qui forment le réseau est séparé du point intermédiaire (223) de celui-ci, et la masse structurelle du rotor (220) étant disposée à proximité de l'axe de rotation (214), ce qui réduit le moment d'inertie dudit rotor.
